# EUROPÄISCHE PATENTSCHRIFT

(11) **EP 0 711 142 B1**
(45) Veröffentlichungstag und Bekanntmachung des Hinweises auf die Patenterteilung: **18.11.1998**
(21) Anmeldenummer: 94924232.5
(22) Anmeldetag: 05.07.1994
(51) Int. Cl.: A61K 7/48, C08F 220/00, A61K 47/32

(54) **VERWENDUNG VON VERNETZTEN COPOLYMERISATEN MONOETHYLENISCH UNGESÄTTIGTER CARBONSÄUREN ALS STABILISATOR IN ÖL-IN-WASSER-EMULSIONEN**
USE OF CROSS-LINKED COPOLYMERS OF MONOETHYLENICALLY UNSATURATED CARBOXYLIC ACIDS AS STABILIZERS IN OIL-IN-WATER EMULSIONS
UTILISATION DE COPOLYMERISATS RETICULES D'ACIDES CARBOXYLIQUES INSATURES MONOETHYLENIQUEMENT COMME STABILISANTS DANS DES EMULSIONS HUILE DANS L'EAU

(30) Priorität: 28.07.1993 DE 4325158
(43) Veröffentlichungstag der Anmeldung: 15.05.1996
(73) Patentinhaber: BASF AKTIENGESELLSCHAFT, 67056 Ludwigshafen (DE)
(72) Erfinder: SCHADE, Christian, D-67061 Ludwigshafen (DE); WESTENFELDER, Horst, D-67435 Neustadt (DE); SPERLING-VIETMEIER, Karin, D-67433 Neustadt (DE); SANNER, Axel, D-67227 Frankenthal (DE); WEKEL, Hans-Ulrich, D-67158 Ellerstadt (DE)
(86) Internationale Anmeldenummer: EP9402194
(87) Internationale Veröffentlichungsnummer: WO9503790

(56) Entgegenhaltungen:
- EP-A- 0 117 410
- EP-A- 0 268 164
- DE-B- 1 138 225
- US-A- 4 419 502

## Beschreibung

Die Erfindung betrifft die Verwendung von vernetzten Copolymerisaten, die durch Fällungspolymerisation von Monomermischungen aus
(a) monoethylenisch ungesättigten C₃-C₈-Carbonsäuren, ihren Anhydriden oder Mischungen aus den genannten Carbonsäuren und Anhydriden,
(b) Verbindungen mit mindestens zwei ethylenisch ungesättigten, nicht konjugierten Doppelbindungen im Molekül als Vernetzer und gegebenenfalls
(c) anderen, mit den Monomeren (a) und (b) copolymerisierbaren monoethylenisch ungesättigten Monomeren in Gegenwart von Radikale bildenden Polymerisationsinitiatoren und bestimmten grenzflächenaktiven Verbindungen hergestellt werden sowie kosmetische oder pharmazeutische Zubereitungen auf Basis von Öl-in-Wasser-Emulsionen, die die obengenannten vernetzten Copolymerisate als Stabilisator enthalten.

Aus der DE-B-1 138 225 ist ein Verfahren zur Herstellung von wasserunlöslichen, wasserquellbaren Mischpolymerisaten durch Fällungspolymerisation von monoethylenisch ungesättigten Carbonsäuren, Monomeren mit mindestens zwei ethylenisch ungesättigten, nicht konjugierten Doppelbindungen im Molekül als Vernetzer und gegebenenfalls anderen wasserunlöslichen monoethylenisch ungesättigten Monomeren in Gegenwart von Radikale bildenden Polymerisationsinitiatoren und von Schutzkolloiden und/oder Emulgatoren bekannt, die sowohl in organischen Lösemitteln als auch in Wasser löslich sind. So erhält man beispielsweise durch Fällungspolymerisation von Acrylsäure und Butandioldiacrylat in 1,2-Dichlorethan in Gegenwart von Polyvinylether ein feines Pulver, das in ammoniakhaltigem Wasser steife Gele bildet, die sich als Salbengrundlagen für Kosmetika eignen. Die vernetzten Polymerisate werden insbesondere als Quell- bzw. Verdickungsmittel verwendet.

Aus der DE-A-2 949 843 ist ein Verfahren zur Herstellung von vernetzten Polymerisaten monoethylenisch ungesättigter Carbonsäuren durch radikalische Fällungspolymerisation der Monomeren in Gegenwart von Radikale bildenden Polymerisationsinitiatoren und Homopolymerisaten des Vinylpyrrolidons als Schutzkolloid bekannt. Die Fällungspolymerisate werden als Verdickungsmittel im Pharma-, Kosmetik-, Papier-, Textil-, Klebstoff- und Dispersionsanstrichsektor verwendet.

Bei dem aus der DE-A-2 833 468 bekannten Verfahren werden beispielsweise Copolymerisate aus Acrylsäure oder Methacrylsäure und Acrylsäureestern oder Methacrylsäureestern gegebenenfalls in Gegenwart von Vernetzungsmitteln der Fällungspolymerisation in Gegenwart von Ethylen-Propylen-Kautschuk unterworfen. Die dabei erhältlichen feinteiligen Polymerisate werden als Verdickungsmittel in Druckpasten, Papierstreichmassen und wäßrigen Anstrichdispersionen eingesetzt.

Aus der US-A-4 419 502 ist bekannt, monoethylenisch ungesättigte Carbonsäuren in Gegenwart von Vernetzern, Radikale bildenden Polymerisationsinitiatoren und Polyoxyethylenalkylethern und/oder Polyoxyethylensorbitanestern in Methylenchlorid zu polymerisieren. Die bei der Fällungspolymerisation mitverwendeten Tenside dienen zur Kontrolle der Teilchengröße der Polymeren, der Verbesserung der Rührbarkeit des Polymerisationsansatzes und zur Verhinderung von Belagbildung im Reaktionsgefäß.

Aus der EP-A-0 268 164 sind lagerstabile, rasch brechende Öl-in-Wasser-Emulsionen bekannt, die ein Copolymerisat aus Acrylsäure mit einem kleineren Anteil an einem langkettigen Alkylacrylat als Stabilisator enthalten. Wie auf Seite 8 dieser Literaturstelle angegeben ist, gelingt es nicht, Öl-in-Wasser-Emulsionen durch Zusatz von Homopolymerisaten der Acrylsäure oder von schwach vernetzten Polyacrylsäuren dauerhaft zu stabilisieren.

Aus der älteren, nicht vorveröffentlichten DE-Anmeldung P 4213283.5 ist die Verwendung von Copolymerisaten aus monoethylenisch ungesättigten Carbonsäuren und langkettigen Verbindungen mit isolierten CC-Mehrfachbindungen und gegebenenfalls weiteren copolymerisierbaren Monomeren und Vernetzern als Verdickungs- oder Dispergiermittel beispielsweise in kosmetischen oder pharmazeutischen Zubereitungen bekannt. Die Copolymerisate werden durch Fällungspolymerisation hergestellt.

Der Erfindung liegt die Aufgabe zugrunde, andere Stabilisatoren für Öl-in-Wasser-Emulsionen zur Verfügung zu stellen.

Die Aufgabe wird erfindungsgemäß gelöst durch Verwendung von vernetzten Polymerisaten, die erhältlich sind durch Fällungspolmerisation von Monomermischungen aus
(a) monoethylenisch ungesättigten C₃- bis C₈-Carbonsäuren, ihren Anhydriden oder Mischungen aus den genannten Carbonsäuren und Anhydriden,
(b) Verbindungen mit mindestens zwei ethylenisch ungesättigten, nicht konjugierten Doppelbindungen im Molekül als Vernetzer und gegebenenfalls
(c) anderen mit den Monomeren (a) und (b) copolymerisierbaren monoethylenisch ungesättigten Monomeren
in Gegenwart von Radikale bildenden Polymerisationsinitiatoren und 0,1 bis 20 Gew.-%, bezogen auf die eingesetzten Monomeren, an gesättigten, nichtionischen grenzflächenaktiven Verbindungem, als Stabilisator in Öl-in-Wasser-Emulsionen in Mengen von 0,01 bis 5 Gew.-%, bezogen auf die Emulsionen.

Gegenstand der Erfindung sind außerdem kosmetische oder pharmazeutische Zubereitungen auf Basis von Öl-in-Wasser-Emulsionen, die als Stabilisator 0,01 bis 5 Gew.-% an vernetzten Polymerisaten enthalten, die erhältlich sind durch Fällungspolymerisation von Monomermischungen aus
(a) monoethylenisch ungesättigen C₃- bis C₈-Carbonsäuren, ihren Anhydriden oder Mischungen aus den genannten Carbonsäuren und Anhydriden,
(b) Verbindungen mit mindestens zwei ethylenisch ungesättigten, nicht konjugierten Doppelbindungen im Molekül als Vernetzer und gegebenenfalls
(c) anderen mit den Monomeren (a) und (b) copolymerisierbaren monoethylenisch ungesättigten Monomeren
in Gegenwart von Radikale bildenden Polymerisationsinitiatoren und 0,1 bis 20 Gew.-%, bezogen auf die eingesetzten Monomeren, an gesättigten, nichtionischen grenzflächenaktiven Verbindungen.

Die in Betracht kommenden vernetzten Copolymerisate werden durch Fällungspolymerisation von Monomermischungen hergestellt. Als Komponente (a) der Monomermischungen verwendet man monoethylenisch ungesättigte C₃- bis C₈-Carbonsäuren, ihre Anhydride oder Mischungen aus den genannten Carbonsäuren und Anhydriden. Geeignete Carbonsäuren sind beispielsweise Acrylsäure, Methacrylsäure, Maleinsäure, Fumarsäure, Itaconsäure, Crotonsäure und 2-Pentensäure. Als Anhydride kommen beispielsweise Methacrylsäureanhydrid, Maleinsäureanhydrid und Itaconsäureanhydrid in Betracht. Von den Monomeren der Gruppe (a) werden Acrylsäure, Methacrylsäure, Maleinsäure, Maleinsäureanhydrid und/oder Methacrylsäureanhydrid bevorzugt eingesetzt. Die Monomere (a) können beispielsweise in den zur Polymerisation eingesetzten Mischungen in Mengen von 50 bis 99,99, vorzugsweise von 80 bis 99,99 Gew.-% enthalten.

Als Monomer der Gruppe (b) kommen Verbindungen mit mindestens zwei ethylenisch ungesättigten, nicht konjugierten Doppelbindungen im Molekül in Betracht. Solche Monomere werden bei Polymerisation üblicherweise als Vernetzer verwendet. Sie erhöhen das Molekulargewicht der entstehenden Copolymerisate. Geeignete Vernetzer sind beispielsweise die Diacrylate oder Dimethacrylate von Glykolen oder Polyalkylenglykolen wie Ethylenglykoldiacrylat, Ethylenglykoldimethacrylat, Diethylenglykolmethacrylat, Diethylenglykoldiacrylat oder Diacrylate oder Dimethacrylate von Polyethylenglykolen mit Molmassen bis 2000, Divinylbenzol, Divinyldioxan, Divinylethylenharnstoff, Diallylweinsäurediamid, Methylenbisacrylamid, mindestens zweifach mit Acrylsäure oder Methacrylsäure veresterte mehrwertige Alkohole wie Trimethylpropan, Pentaerythrit, Butandiol-1,4, Hexandiol-1,6 und Sorbit, Trivinylcyclohexan, Triallyltriazintrion, Allylester der Acrylsäure und der Methacrylsäure sowie Allylether von mehrwertigen Alkoholen, z.B. die Di- und Triallylether von Trimethylolpropan, Pentaerythrit, Sorbit und Saccharose. Bevorzugt eingesetzte Vernetzer sind Pentaerythrittriallylether, Diacrylate oder Dimethacrylate von Glykolen oder Polyethylenglykolen mit Molmassen bis zu 2000, Pentaallylsaccharose, Methacrylsäureallylester, Trimethylpropandiallylether und/oder Methylenbisacrylamid. Die Mengen an Vernetzer in der Monomermischung betragen vorzugsweise 0,01 bis 20 Gew.%. In den meisten Fällen enthalten die zur Polymerisation eingesetzten Monomermischungen 0,1 bis 2 Gew.-% an Vernetzer, wobei auch Mischungen verschiedener Vernetzer eingesetzt werden können.

Geeignete andere, mit den Monomeren (a) und (b) copolymerisierbare monoethylenisch ungesättigte Monomere der Gruppe (c) sind beispielsweise N-Vinylpyrrolidon, N-Vinylcaprolactam, C₁- bis C₁₈-Alkyl(meth)acrylate, beispielsweise Methylacrylat, Methylmethacrylat, Ethylacrylat, Ethylmethacrylat, Propylacrylat, Propylmethacrylat, n-Butylacrylat, n-Butylmethacrylat, Isobutylacrylat, Isobutylmethacrylat, 2-Ethylhexylacrylat, 2-Ethylhexylmethacrylat, Stearylacrylat oder Stearylmethacrylat, Acrylamid, Methacrylamid, N-(C₁-bis C₈-Alkyl)acrylamide oder -methacrylamide wie N,N-Dimethylacrylamid, N,N-Dimethylmethacrylamid, N-tert.Butylacrylamid, N-tert.Butylmethacrylamid, N-tert.-Octylacrylamid oder N-tert.Octylmethacrylamid, Vinylester von gesättigten C₁- bis C₈-Carbonsäuren, wie Vinylacetat, Vinylpropionat, Vinylbutyrat oder Vinylstearat, Styrol, Phenoxyethylacrylat, Hydroxyalkylenmonoacrylester und Hydroxyalkylenmonomethacrylester mit jeweils 2 bis 6 Kohlenstoffatomen in der Alkylenkette oder Acrylsäureester und Methacrylsäureester von methoxylierten C₁- bis C₁₈-Alkoholen, wobei pro Mol Alkohol 2 bis 25 Ethylenoxideinheiten angelagert sind. Falls die Verbindungen der Gruppe (c) zur Modifizierung der Copolymerisate eingesetzt werden, beträgt ihr Anteil in der Monomermischung bis zu 49,89, vorzugsweise 0 bis 19,09 Gew.-%. Bevorzugt verwendete Monomere der Gruppe (c) sind N-Vinylpyrrolidon, C₁-C₁₈-Alkyl(meth)acrylate, Styrol, Hydroxyethyl(meth)acrylat und Ethyldiglycolacrylat.

Die erfindungsgemäß zu verwendenden vernetzten Copolymerisate werden in Gegenwart von speziellen grenzflächenaktiven Verbindungen hergestellt. Eine Gruppe dieser grenzflächenaktiven Verbindungen sind gesättigte, nicht ionische Tenside, wie Ester von Zuckern oder Zuckerderivaten, wie Saccharoseester, Mannoseester, Xyloseester oder Sorbitanester, Ester und Ether von Glycerin, Polyglycerin oder Glycerin-Zucker-Kondensate, Ceramide und Glycosyl-Ceramide, Fettsäurealkanolamide wie Fettsäureethanolamide, Fettsäureisopropanolamide, Fettsäurediethanolamide, Fettsäurepolydiethanolamide, N-Alkylpyrrolidonderivate, Pyrrolidon-5-carbonsäure-alkylester, Zitronen- und Weinsäurester, C₁- bis C₁₈-Alkyl(poly)glycoside, Hydroxyalkylpolyglycoside, Fettsäureester von Polyhydroxyverbindungen wie Trimethylolpropan, Erythrit, Pentaerythrit, Neopentyldiglycol, Triethanolamin oder daraus abgeleitete Kondensate, Alkoxylate, insbesondere die Additionsprodukte von Ethylenoxid und/oder Propylenoxd an die oben aufgeführten Verbindungen sowie an Oxoalkohole, C₈- bis C₃₀-Alkohole, Alkylphenole, Fettsäureanamide, Fettamine, Fettsäuren und Derivate wie Hydroxycarbonsäure, wobei die Polyalkylenoxidketten einseitig oder beidseitig modifiziert sein können. Bei beidseitiger Modifizierung können die modifizierenden Anteile gleich oder verschieden sein und z.B. zu einem Teil auch eine C₁- bis C₄-Etherfunktion darstellen.

Polymere Tenside, die Ethylenoxid- und/oder Propylenoxid-Einheiten als hydrophilen Teil des Moleküls enthalten, sind unvernetzt und haben Molmassen von 500 bis 100000, vorzugsweise 700 bis 20000. Die polymeren Tenside können neben mindestens einem hydrophilen Block mindestens einen hydrophoben Block enthalten oder sie sind aus einer hydrophilen Kette mit kammartig angeordneten hydrophoben Ästen aufgebaut. Der hydrophile Teil der polymeren Tenside wird von Homopolymerisaten aus Ethylenoxid oder Propylenoxid oder von Blockcopolymerisaten aus Ethylenoxid und Propylenoxid sowie von Block- und Kammpolymerisaten mit Blöcken aus Polyethylenoxid, Polypropylenoxid oder Polyco(ethylenoxid, Propylenoxid) gebildet, während der hydrophobe Teil der polymeren Tenside von Blöcken aus Polystyrolen, Polyalkyl(meth)acrylaten, Silikonölen, Polyhydroxyfettsäuren, Polyamidoaminen, Polyisobutylen oder Polytetrahydrofuranen besteht. Es können auch allgemeine Polymere, die über mindestens einer Aminogruppe, eine mit Basen deprotonierbare Hydroxylgruppe oder eine anionische Gruppe verfügen und eine Molmasse von 100 bis 5000 haben, mit Ethylenoxid, Propylenoxid oder Mischungen daraus zu geeigneten polymeren Tensiden umgesetzt werden.

Weitere grenzflächenaktive Verbindungen sind Sorbitanester, Saccharoseester oder Glycerinester von gesättigten C₈- bis C₃₀-Carbonsäuren oder Alkoxylierungsprodukte dieser Ester. Die vorstehend genannten Ester leiten sich vorzugsweise von C₁₂- bis C₂₂-Carbonsäuren ab. Unter Alkoxylierungsprodukten sollen vorzugsweise die Additionsprodukte von Ethylenoxid an die Ester verstanden werden. Pro Mol der in Betracht kommenden Ester können bis zu 80 Mol Ethylenoxid addiert werden. Daneben eignen sich auch Additionsprodukte von Ethylenimin und Propylenoxid und/oder von Butylenoxiden an die Ester als grenzflächenaktive Verbindungen.

Weitere gesättigte, nichtionische grenzflächenaktive Verbindungen sind hydrophob modifizierte Cellulose und/oder Stärke wie beispielsweise Ethylcellulosen, Hydroxypropyl-methyl-Cellulosen, Methylcellulosen, Hydroxypropylcellulosen oder Cellulosetriacetat.

Von den obengenannten gesättigten, nichtionischen grenzflächenaktiven Verbindungen werden vorzugsweise Saccharoseester, Sorbitanester, Glycerinester, Alkyl(poly)glycoside, Additionsprodukte von Ethylenoxid an die vorstehend genannten Verbindungen und Additionsprodukte von Ethylenoxid an C₁₂- bis C₂₂-Alkohole eingesetzt, wobei die Verwendung von Sorbitanstearat, Sorbitanmonolaurat und hydrierten Ricinusölethoxylaten besonders bevorzugt ist.

Die oben beschriebenen Tenside wie auch die polymeren Tenside und modifizierten Cellulosen und Stärken sind oberflächenaktive Verbindungen. Sie bestehen aus einem hydrophoben und einem hydrophilen Molekül. Soweit sie in Wasser ausreichend löslich sind, besitzen sie in 1 gew.-%iger wäßriger Lösung eine gegen Luft gemessene Oberflächenspannung von weniger als 66 mN/m bei 20°C.

Die oben beschriebenen oberflächenaktiven Verbindungen werden bei der Fällungspolymerisation in Mengen von 0,1 bis 20, vorzugsweise 0,25 bis 10 Gew.-%, bezogen auf die eingesetzten Monomeren, eingesetzt.

Die Fällungspolymerisation wird üblicherweise in einem Lösemittel durchgeführt, in dem die Monomeren löslich und die entstehenden Polymerisate unlöslich sind. Als Lösemittel sind beispielsweise aromatische und gesättigte aliphatische Kohlenwasserstoffe geeignet. Beispiele für aromatische Kohlenwasserstoffe sind Benzol, Toluol, Xylol und Isopropylbenzol. Die gesättigten aliphatischen Kohlenwasserstoffe haben vorzugsweise 5 bis 12 Kohlenstoffatome. Geeignet sind Pentan, n-Hexan, Cyclohexan, Octan und Isooctan. Die Fällungspolymerisation kann auch in halogenierten gesättigten aliphatischen Kohlenwasserstoffen durchgeführt werden, wie 1,1,1-Trichlorethan oder Methylenchlorid. Als Reaktionsmedium eignen sich außerdem Ether, C₂-C₆-Alkylester der Ameisensäure oder der Essigsäure, Ketone mit 3 bis 6 Kohlenstoffatomen, flüssiges oder überkritisches Kohlendioxid. Geeignete Ether sind beispielsweise tert.-Butylmethylether oder Isobutylmethylether. Die Alkylester der Ameisensäure oder Essigsäure leiten sich vorzugsweise von gesättigten Alkoholen mit. 2 bis 6 Kohlenstoffatomen ab, z.B. Ameisensäureethylester, Essigsäuremethylester oder Essigsäureethylester. Geeignete Ketone sind beispielsweise Aceton und Methylethylketon. Die Verdünnungsmittel können alleine oder in Mischung untereinander eingesetzt werden. Vorzugsweise werden als Verdünnungsmittel bei der Fällungspolymerisation gesättigte aliphatische Kohlenwasserstoffe mit 5 bis 8 C-Atomen im Molekül eingesetzt, die geradkettig oder verzweigt, cyclisch oder dicyclisch sein können. Besonders bevorzugt wird Cyclohexan als Lösemittel bei der Fällungspolymerisation eingesetzt. Die Menge an Lösungemittel wird so gewählt, daß die Reaktionsmischung während der Polymerisation gerührt werden kann. Der Feststoffgehalt der Mischung liegt nach der Polymerisation vorzugsweise in dem Bereich von 10 bis 40 Gew.-%.

Das Molekulargewicht der Copolymerisate kann gewünschtenfalls durch Zugabe von Reglern zur polymerisierenden Mischung erniedrigt werden. Geeignete Regler sind beispielsweise Mercaptoverbindungen wie Dodecylmercaptan, Thioethanol, Thioglykolsäure oder Mercaptopropionsäure. Falls Regler mitverwendet werden, werden sie in Mengen von 0,1 bis 5 Gew.-%, bezogen auf die eingesetzten Monomeren, verwendet.

Die Copolymerisation erfolgt in Gegenwart von Radikale bildenden Polymerisationsinitiatoren. Geeignete Verbindungen dieser Art sind Azo- oder Peroxoverbindungen, z. B. Diacylperoxide wie Dilauroylperoxid, Didecanoylperoxid und Dioctanoylperoxid oder Perester wie tert.-Butylperoctanoat, tert.-Butylperpivalat, tert.-Amylperpivalat oder tert.-Butylperneodecanoat oder Azoverbindungen wie Dimethyl-2,2'-azobis(isobutyrat), 2,2'-Azobis (isobutyronitril), 2,2'-Azobis(2-methylbutyronitril) oder 2,2'-Azobis(2,4-dimethylvaleronitril). Die Inititatoren werden in den bei Fällungspolymerisationen üblichen Mengen eingesetzt, z. B. in Mengen von 0,05 bis 5 Gew.-%, bezogen auf die Monomeren. Falls bei der Fällungspolymerisation Wasser und/oder Basen mitverwendet werden, dann nur in solchen Mengen, daß die Mischung aller Komponenten vor dem Beginn der Polymerisation noch homogen erscheint.

Die Fällungspolymerisation wird üblicherweise unter einer Inertgasatmosphäre durchgeführt. Die Copolymerisation kann beispielsweise in der Art durchgeführt werden, daß man alle Komponenten, die während der Polymerisation anwesend sind, in einem Polymerisationsgefäß vorlegt und die Reaktion startet und das Reaktionsgemisch gegebenenfalls kühlt, um die Polymerisationstemperatur zu kontrollieren. Man kann jedoch auch so vorgehen, daß man nur einen Teil der zu polymerisierenden Komponenten vorlegt, die Polymerisation startet und den Rest der zu polymerisierenden Mischung je nach Fortschritt der Polymerisation kontinuierlich oder absatzweise zudosiert. Man kann jedoch auch so verfahren, daß man zunächst das Verdünnungsmittel zusammen mit einem Tensid vorlegt und die Monomeren und den Polymerisationsinitiator in separaten Zuläufen in die Vorlage kontinuierlich oder absatzweise einbringt.

Die Temperatur während der Polymerisation liegt im allgemeinen zwischen 40 und 160, vorzugsweise 50 bis 120°C. Sie kann während der Reaktion nach einem Programm unterschiedlich gesteuert werden. Die Polymerisation wird vorzugsweise unter Atmosphärendruck durchgeführt, kann jedoch auch unter vermindertem oder erhöhtem Druck vorgenommen werden. Sofern die Polymerisationstemperatur oberhalb des Siedepunkts des inerten Verdünnungsmittels liegt, wird die Polymerisation in druckdichten Apparaturen bei Drücken bis zu 8 bar durchgeführt. Falls Kohlendioxid als inertes Verdünnungsmittel eingesetzt wird, wird die Polymerisation üblicherweise in einem Autoklaven oberhalb der kritischen Temperatur von Kohlendioxid durchgeführt. Die Drücke liegen dann oberhalb von 73 bar.

Der Polymerisationsprozeß wird vorzugsweise so gesteuert, daß das Copolymerisat in Form eines feinteiligen Pulvers anfällt. Die mittlere Teilchengröße des Polymerisatpulvers beträgt 0,1 bis 500, vorzugsweise 0,5 bis 200 µm. Nach der Polymerisation wird das vernetzte Copolymerisat von den übrigen Bestandteilen der Reaktionsmischung abgetrennt, beispielsweise abfiltriert, abdekantiert oder abzentrifugiert. Das so erhaltene Pulver kann gegebenenfalls weiteren geeigneten Trenn-, Wasch-, Trocknungs- oder Mahlverfahren unterworfen werden.

Von besonderem Interesse sind solche Fällungspolymerisate, die durch Copolymerisieren von Monomermischungen aus
a) 80 bis 99,99 Gew.-% Acrylsäure, Methacrylsäure, Maleinsäure, Maleinsäureanhydrid und/oder Methacrylsäureanhydrid und
b) 0,01 bis 20 Gew.-% Pentaearythittriallylether, Diacrylaten oder Dimethacrylaten von Glykolen oder Polyethylenglykolen mit Molmassen bis zu 2000, Pentaallylsaccarose, Methacrylsäureallylester, Trimethylolpropandiallylether und/oder Methylenbisacrylamid
erhältlich sind. Die entstehenden Copolymerisate sind vernetzt und in Wasser nicht löslich. Sie quellen jedoch in Wasser.

Die oben beschriebenen Copolymerisate werden als Stabilisator in Öl-in-Wasser-Emulsionen in Mengen von 0,01 bis 5 Gew.-%, bezogen auf die Emulsionen, verwendet. Sie eignen sich zum Stabilisieren sämtlicher Öl-in-Wasser-Emulsionen, z. B. von Öl in Wasser-Polymeremulsionen, Entschäumern auf Basis von Öl-in-Wasser-Emulsionen, Druckpasten für den Textildruck, Anstrichfarben, Reinigungsmittelformulierungen, Bohrlochspülungen, Flüssigwaschmitteln und insbesondere zur Stabilisierung von kosmetischen oder pharmazeutischen Zubereitungen auf Basis von Öl-in-Wasser-Emulsionen.

Um eine daverhafte Stabilisation von Öl-in-Wasser-Emulsion zu erzielen, wird das dispergierte Polymer mit einer Base ausreichend neutralisiert. Geeignete Basen sind z.B. Alkalibasen wie Alkalimetallhydroxide und -carbonate, beispielsweise NaOH, KOH und Natrium- und Kaliumcarbonat, Ammoniak und organische Amine, Pyridine und Amidine oder Mischungen daraus. Bei der Neutralisation mit Hilfe organischer Amine verwendet man vorzugsweise Alkanolamine aus der Reihe der Mono- Di- oder Trialkanolamine mit 2 bis 5 C-Atomen im Alkanolrest wie Mono-, Di- oder Triethanolamin, Mono-, Di- oder Tri(iso)propanolamin oder 2-Amino-2-methylpropanol, Alkandiolamine mit 2 bis 4 C-Atomen im Alkandiolrest wie 2-Amino-2-methyl-1,3-propandiol oder 2-Amino-2-ethyl-1,3-propandiol, Alkanpolyolamine wie Tetrahydroxypropylethylendiamin, Tris(hydroxmethyl)aminomethan oder N,N,N',N'-Tetrakis(2-hydroxypropyl)ethylendiamin, Alkylamine wie Di(2-ethylhexyl)amin, Triamylamin oder Dodecylamin und Aminoether wie Morpholin.

Die kosmetischen oder pharmazeutischen Zuberetungen können dabei als Öl alle hierfür üblicherweise verwendbaren Öle enthalten. Die Gesamtmenge der Ölphase in der Emulsion kann dabei bis zu 80 Gew.-% betragen. Der Anteil der Ölphase in den kosmetischen oder pharmazeutischen Zubereitungen liegt bevorzugt bei 10 bis 50 Gew.-%. Die schwach vernetzten Copolymerisate werden vorzugsweise zum Stabilisieren in Cremes oder Lotionen angewandt. Daneben eignen sie sich gut zum Verdicken wäßriger Systeme oder Ausbildung verdickter Gele, nachdem das dispergierte Copolymerisat durch Zugabe einer Base, z. B. Triethanolamin, Natronlauge, Kalilauge, 2-Amino-2-methyl-1-propanol, 2-Amino-2-methyl-1,3-propandiol, Diisopropanolamin oder Tetrahydroxypropylethylendiamin ausreichend neutralisiert worden ist.

Im Gegensatz zu schwach vernetzten Homopolymerisaten der Acrylsäure gelingt es, mit den erfindungsgemäß zu verwendenden, schwach vernetzten Copolymerisaten Öl-in-Wasser-Emulsionen dauerhaft zu stabilisieren. Die bevorzugt zum Einsatz gelangenden Mengen an vernetzten Copolymerisaten, die durch Fällungspolymerisation hergestellt werden, beträgt 0,05 bis 2 Gew.-%, bezogen auf die Emulsionen.

### Beispiele

### Bestimmung der Gelviskosität

In einem 300 ml-fassenden Becherglas werden 1 g eines vernetzten Copolymers und 200 g Wasser eingewogen und homogen verrührt. Danach setzt man 1 ml Triethanolamin zu und rührt solange bis eine homogene Mischung entsteht. Danach bestimmt man mit einem Haake-Handviskosimeter VT-02 mit der Spindel 1 die Viskosität des Gemisches bei 20°C und 60 U/min.

### Polymer 1

In einem 3000 ml fassenden Kolben, der mit einem Rührer und einer Apparatur für das Arbeiten unter Schutzgas ausgestattet war, wurden 1320 ml Cyclohexan, 50 g Acrylsäure, 1,5 g Pentaerythrittriallylether, 7,5 g Sorbitanstearat und 80 mg 2,2'-Azobis(2-methyl)-butyronitril) vorgelegt und unter Rühren im Stickstoff-Strom auf 80°C erhitzt. Nach dem Erreichen dieser Temperatur wurde ein Zulauf aus 200 g Acrylsäure während 2 Stunden und ein weiterer Zulauf aus 80 ml Cyclohexan und 320 mg 2,2'-Azobis(2-methyl-butyronitril) während 3 Stunden zugetropft. Nach Beendigung der Zugabe des Polymerisationsinitiators wurde das Reaktionsgemisch noch 3 Stunden bei 80°C gerührt. Das Produkt wurde anschließend über eine Filternutsche abgesaugt und im Vakuumtrockenschrank bei 50°C für 8 Stunden getrocknet. Man erhielt 251 g eines weißen Polymerpulvers mit einer Gelviskosität von 7 Pa·s.

### Polymer 2

In einem 3000 ml fassenden Kolben, der mit einem Rührer und einer Apparatur für das Arbeiten unter Schutzgas ausgestattet war, wurden 1320 ml Cyclohexan, 50 g Acrylsäure, 1,2 g Pentaerythrittriallylether, 1,5 g Triglyceryldistearat und 80 mg 2,2'-Azobis(2-methyl-butyronitril) vorgelegt und unter Rühren im Stickstoff-Strom auf 80°C erhitzt. Nach dem Erreichen dieser Temperatur wurde ein Zulauf aus 200 g Acrylsäure und 6 g Triglyceryldistearat während 2 Stunden und ein weiterer Zulauf aus 80 ml Cyclohexan und 320 mg 2,2'-Azobis(2-methyl-butyronitril) während 3 Stunden zugetropft. Nach Beendigung der Zugabe des Polymerisationsinitiators wurde das Reaktionsgemisch noch 3 Stunden bei 80°C gerührt. Das Produkt wurde anschließend über eine Filternutsche abgesaugt und im Vakuumtrockenschrank bei 50°C für 8 Stunden getrocknet. Man erhielt 251 g eines weißen Polymerpulvers mit einer Gelviskosität von 7,5 Pa·s.

### Polymer 3

In einem 3000 ml fassenden Kolben, der mit einem Rührer und einer Apparatur für das Arbeiten unter Schutzgas ausgestattet war, wurden 1320 ml Cyclohexan, 50 g Acrylsäure, 1,5 g Pentaerythrittriallylether 80 mg 2,2'-Azobis(2-methyl-butyronitril) und 7,5 g eines hydrierten Ricinusölethoxylats mit 47 Ethylenoxid-Einheiten im Molekül vorgelegt und unter Rühren im Stickstoff-Strom auf 80°C erhitzt. Nach dem Erreichen dieser Temperatur wurde ein Zulauf aus 200 g Acrylsäure während 2-Stunden und ein weiterer Zulauf aus 80 ml Cyclohexan und 320 mg 2,2'-Azobis(2-methyl-butyroritril) während 3 Stunden zugetropft. Nach Beendigung der Zugabe des Polymerisationsinitiators wurde das Reaktionsgemisch noch 3 Stunden bei 80°C gerührt. Das Produkt wurde anschließend über eine Filternutsche abgesaugt und im Vakuumtrockenschrank bei 50°C für 8 Stunden getrocknet. Man erhielt 234 g eines weißen Polymerpulvers mit einer Gelviskosität von 11 Pa·s.

### Polymer 4

In Analogie zur Herstellung von Polymer 3 wurde ein Polymer aus 250 g Acrylsäure, 1,5 g Pentaerythrittriallylether und 7,5 g Saccharosestearat hergestellt. Man erhielt 255 g eines weißen Polymerpulvers mit einer Gelviskosität von 7 Pa·s.

### Polymer 5

In Analogie zur Herstellung von Polymer 3 wurde ein Polymer aus 250 g Acrylsäure, 1,2 g Pentaerythrittriallylether und 7,5 g eines Celluloseethers mit einem Substitutionsgrad von 46 % und einer Viskosität von 0,1 Pa·s [gemessen in 5 %iger Lösung in Toluol/Ethanol = 4 : 1 (v/v) bei 25°C, Ubbelohde-Viskosimeter] hergestellt. Man erhielt 243 g eines weißen Polymerpulvers mit einer Gelviskosität von 10,5 Pa·s.

### Polymer 6

In Analogie zur Herstellung von Polymer 3 wurde ein Polymer aus 250 g Acrylsäure, 1,5 g Pentaerythrittylether und 7,5 g Cetearylpolyglycosid hergestellt. Man erhielt 250 g eines weißen Polymerpulvers mit einer Gelviskosität von 8,5 Pa·s.

### Polymer 7

In Analogie zur Herstellung von Polymer 3 wurde ein Polymer aus 250 g Acrylsäure, 1,5 g Methacrylsäureallylester und 7,5 g eines Ethylenoxid-Propylenoxid-Blockcopolymers mit einen Molekulargewicht von 2000 und einem Trübungspunkt in Wasser von 23°C hergestellt. Man erhielt 247 g eines weißen Polymerpulvers mit einer Gelviskosität von 7 Pa·s.

### Polymer 8

In Analogie zur Herstellung von Polymer 3 wurde ein Polymer aus 250 g Acrylsäure, 1,5 g Pentaerythrittriallylether und 7,5 g eines Polystyrol-Polyethylenoxid-Blockcopolymers mit einem Molekulargewicht von 2000 und einem Styrol/Ethylenoxid-Verhältnis von 1 : 1 hergestellt. Man erhielt 253 g eines weißen Polymerpulvers mit einer Gelviskosität von 10 Pa·s.

### Polymer 9

In Analogie zur Herstellung von Polymer 3 wurde ein Polymer aus 250 g Acrylsäure, 1,2 g Pentaerythrittallylether und 7,5 g Sorbitanmonolaurat hergestellt. Man erhielt 248 g eines weißen Polymerpulvers mit einer Gelviskosität von 8 Pa·s.

### Polymer 10

In Analogie zur Herstellung von Polymer 3 wurde ein Polymer aus 250 g Acrylsäure, 1,2 g Pentaerythrittallylether und 12,5 g eines Myristylalkohols, der mit 2,5 Ethylenoxid- und 5 Propylenoxid-Einheiten je Molekül ungesetzt worden war, hergestellt. Man erhielt 258 g eines weißen Polymerpulvers mit einer Gelviskosität von 9 Pa·s.

### Polymer 11

In Analogie zur Herstellung von Polymer 3 wurde ein Polymer aus 250 g Acrylsäure, 1,2 g Pentaerythrittallylether und 7,5 g Kokosfettsäurediethanolamid hergestellt. Man erhielt 234 g eines weißen Polymerpulvers mit einer Gelviskosität von 5 Pa·s.

### Polymer 12

In Analogie zur Herstellung von Polymer 3 wurde ein Polymer aus 250 g Acrylsäure, 1,5 g Pentaerythrittallylether und 7,5 g eines polyoxyethylen-sobitan-monolaurats mit einem Ethoxylierungsgrad von 20 hergestellt. Man erhielt 252 g eines weißen Polymerpulvers mit einer Gelviskosität von 8 Pa·s.

### Vergleichsbeispiel 1

In einem 3000 ml fassenden Kolben, der mit einem Rührer und einer Apparatur für das Arbeiten unter Schutzgas ausgestattet war, wurden 1320 ml Cyclohexan, 50 g Acrylsäure, 0,3 g Pentaerythrittriallylether und 80 mg 2,2'-Azobis(2-methyl-butyronitril) vorgelegt und unter Rühren im Stickstoff-Strom auf 80°C erhitzt. Nach dem Erreichen dieser Temperatur wurde ein Zulauf aus 200 g Acrylsäure und 1,2 g Pentaerythrittallylether während 2 Stunden und ein weiterer Zulauf aus 80 ml Cyclohexan und 320 mg 2,2'-Azobis(2-methyl-butyronitril) während 3 Stunden zugetropft. Nach Beendigung der Zugabe des Polymerisationsinitiators wurde das Reaktionsgemisch noch 3 Stunden bei 80°C gerührt. Das Produkt wurde anschließend über eine Filternutsche abgesaugt und im Vakuumtrockenschrank bei 50°C für 8 Stunden getrocknet. Man erhielt 247 g eines weißen Polymerpulvers mit einer Gelviskosität von 13 Pa·s.

### Herstellung und Beurteilung von Paraffinöl-Wasser-Emulsionen

In einem 300 ml fassenden Gefäß werden 0,4 g Polymer und 30 g Paraffinöl eingewogen und homogen verrührt. Danach werden 103,6 ml Wasser zugesetzt und für 30 min verrührt. Schließlich setzt man 0,4 g Triethanolamin zu und verrührt zu einer voremulgierten Phase. Anschließend wird die Mischung mit einem Dispergieraggregat umgesetzt, bis eine homogene weiße Emulsion mit einer mittleren Teilchengröße der emulgierten Ölphase von weniger als 50 µm entstanden ist. Die Emulsion wird in einen 100 ml Meßzylinder gefüllt, mit einem Stopfen verschlossen und für eine Woche bei 25°C gelagert.

### Emulsionen 1 - 5 (Vergleich gemäß Stand der Technik)

Es wurden Emulsionen mit dem Polymer aus Vergleichsbespiel 1 und der unten angegebenen Menge eines Tensids nach der gegebenen Vorschrift hergestellt.
- Emulsion 1:: 12 mg Sorbitanstearat
- Emulsion 2:: 12 mg hydriertes Ricinusölethoxylat mit 47 Ethylenoxid-Einheiten im Molekül
- Emulsion 3:: 12 mg Cetearypolyglycosid
- Emulsion 4:: 12 mg Sorbitan-monolaurat
- Emulsion 5:: 50 mg Sorbitan-monolaurat

Alle Emulsionen begannen sich nach spätestens 12 - 24 Stunden zu trennen und zeigten nach spätestens 60 h zwei getrennte Phasen.

### Beispiele 1 - 11

Nach der oben angegebenen Vorschrift wurden Emulsionen mit den Polymeren 1 - 11 hergestellt. Alle Emulsionen waren nach 170 Stunden noch stabil und zeigten keine Tendenz zur Trennung.

### Beispiele 12 - 18

Mit Polymer 3 wurden nach der gegebenen Vorschrift Öl-in-Wasser-Emulsionen hergestellt, wobei anstelle des Paraffinöls folgende Öle eingesetzt wurden:
- Beispiel 12:: Erdnußöl
- Beispiel 13:: Jojobaöl
- Beispiel 14:: Caprinsäure-triglycerid
- Beispiel 15:: lineares Polydimethylsiloxan, Viskosität 0,35 Pa·s
- Beispiel 16:: Isostearinsäure
- Beispiel 17:: Ölsäuredecylester

### Beispiel 18: Ethylhexansäureester eines C₁₆/C₁₈-Fettalkohols

Alle Emulsionen waren nach 170 Stunden noch stabil und zeigten keine Tendenz zur Trennung.

### Beispiele 19 - 22

Mit Polymer 8 wurden Paraffinöl-Wasser-Emulsionen nah der angegebenen Vorschrift hergestellt, wobei die verwendete Ölmenge durch die angegebene Menge ersetzt wurde:
- Beispiel 19:: 15 g Paraffinöl
- Beispiel 20:: 45 g Paraffinöl

Analog wurde eine Emulsion mit Polymer 8 nach der angegebenen Vorschrift hergestellt, wobei die verwendete Polymermenge durch die angegebene Menge ersetzt wurde:
- Beispiel 21:: 0,27 g
- Beispiel 22:: 0,53 g

Alle Emulsionen waren nach 170 Stunden noch stabil und zeigten keine Tendenz zur Trennung.

## Patentansprüche

1. Verwendung von vernetzten Copolymerisaten, die erhältlich sind durch Fällungspolymerisation von Monomermischungen aus
(a) monoethylenisch ungesättigten C₃- bis C₈-Carbonsäuren, ihren Anhydriden oder Mischungen aus den genannten Carbonsäuren und Anhydriden,
(b) Verbindungen mit mindestens 2 ethylenisch ungesättigten, nicht konjugierten Doppelbindungen im Molekül als Vernetzer und gegebenenfalls
(c) anderen, mit den Monomeren (a) und (b) copolymerisierbaren monoethylenisch ungesättigten Monomeren
in Gegenwart von Radikale bildenden Polymerisationsinitiatoren und 0,1 bis 20 Gew.-%, bezogen auf die eingesetzten Monomeren, an gesättigten, nichtionischen grenzflächenaktiven Verbindungen, als Stabilisator in Öl-in-Wasser-Emulsionen in Mengen von 0,01 bis 5 Gew.-%, bezogen auf die Emulsionen.

2. Verwendung nach Anspruch 1, dadurch gekennzeichnet, daß man als gesättigte, nichtionische grenzflächenaktive Verbindungen gesättigte, nichtionische Tenside einsetzt.

3. Verwendung nach Anspruch 1, dadurch gekennzeichnet, daß man als gesättigte, nichtionische grenzflächenaktive Verbindungen Sorbitanester, Saccharoseester oder Glycerinester von gesättigten C₈- bis C₃₀-Carbonsäuren oder Alkoxylierungsprodukte dieser Ester einsetzt.

4. Verwendung nach Anspruch 1, dadurch gekennzeichnet, daß man als gesättigte, nichtionische grenzflächenaktive Verbindungen hydrophob modifizierte Cellulose und/oder hydrophob modifizierte Stärke einsetzt.

5. Verwendung nach einem der Ansprüche 1 bis 4, dadurch gekennzeichnet, daß Monomermischungen aus
(a) 80 bis 99,99 Gew.-% Acrylsäure, Methacrylsäure, Maleinsäure, Maleinsäureanhydrid und/oder Methacrylsäureanhydrid, und
(b) 0,01 bis 20 Gew.-% Pentaerythrittriallylether, Diacrylaten oder Dimethacrylaten von Glykolen oder Polyethylenglykolen mit Molmassen bis zu 2000, Pentaallylsaccharose, Methacrylsäureallylester, Trimethylolpropandiallylether und/oder Methylenbisacrylamid der Fällungspolymerisation unterworfen werden.

6. Kosmetische oder pharmazeutische Zubereitungen auf Basis von Öl-in-Wasser-Emulsionen, dadurch gekennzeichnet, daß sie als Stabilisator 0,01 bis 5 Gew.-% an vernetzten Polymerisaten enthalten, die erhältlich sind durch Fällungspolymerisation von Monomermischungen aus
(a) monoethylenisch ungesättigten C₃- bis C₈-Carbonsäuren, ihren Anhydriden oder Mischungen aus den genannten Carbonsäuren und Anhydriden,
(b) Verbindungen mit mindestens 2 ethylenisch ungesättigten, nicht konjugierten Doppelbindungen im Molekül als Vernetzer und gegebenenfalls
(c) anderen, mit den Monomeren (a) und (b) copolymerisierbaren monoethylenisch ungesättigten Monomeren
in Gegenwart von Radikale bildenden Polymerisationsinitiatoren und 0,1 bis 20 Gew.-%, bezogen auf die eingesetzten Monomeren, an gesättigten, nichtionischen grenzflächenaktiven Verbindungen.

## Claims

1. The use of crosslinked copolymers obtainable by precipitation polymerization of monomer mixtures comprising
(a) monoethylenically unsaturated C₃-C₈-carboxylic acids, their anhydrides or mixtures of said carboxylic acids and anhydrides,
(b) compounds with at least 2 non-conjugated ethylenic double bonds in the molecule as crosslinkers and, where appropriate,
(c) other monoethylenically unsaturated monomers which are copolymerizable with monomers (a) and (b),
in the presence of free-radical polymerization initiators and from 0.1 to 20% by weight, based on the monomers used, of saturated, nonionic surface-active compounds, as stabilizer in oil-in-water emulsions in amounts of from 0.01 to 5% of the weight of the emulsions.

2. The use as claimed in claim 1, wherein saturated, nonionic surfactants are used as saturated, nonionic surface-active compounds.

3. The use as claimed in claim 1, wherein sorbitan esters, sucrose esters or glycerol esters of saturated C₈-C₃₀-carboxylic acids or alkoxylation products of these esters are used as saturated, nonionic surface-active compounds.

4. The use as claimed in claim 1, wherein hydrophobically modified cellulose and/or hydrophobically modified starch is used as saturated, nonionic surface-active compounds.

5. The use as claimed in any of claims 1 to 4, wherein monomer mixtures comprising
a) 80-99.99% by weight of acrylic acid, methacrylic acid, maleic acid, maleic anhydride and/or methacrylic anhydride and
b) 0.01-20% by weight of pentaerythritol triallyl ether, diacrylates or dimethacrylates of glycols or polyethylene glycols with molecular weights of up to 2000, pentaallylsucrose, allyl methacrylate, trimethylolpropane diallyl ether and/or methylenebisacrylamide are subjected to precipitation polymerization.

6. A cosmetic or pharmaceutical formulation based on an oil-in-water emulsion, wherein it contains as stabilizer from 0.01 to 5% by weight of crosslinked polymers obtainable by precipitation polymerization of monomer mixtures comprising
(a) monoethylenically unsaturated C₃-C₈-carboxylic acids, their anhydrides or mixtures of said carboxylic acids and anhydrides,
(b) compounds with at least 2 non-conjugated ethylenic double bonds in the molecule as crosslinkers and, where appropriate,
(c) other monoethylenically unsaturated monomers which are copolymerizable with monomers (a) and (b),
in the presence of free-radical polymerization initiators and from 0.1 to 20% by weight, based on the monomers used, of saturated, nonionic surface-active compounds.

## Revendications

1. Utilisation de copolymères réticulés, qui peuvent être obtenus par polymérisation par précipitation de mélanges de monomères de
(a) des acides carboxyliques en C₃-C₈ à insaturation monoéthylénique, leurs anhydrides ou des mélanges des acides carboxyliques et des anhydrides indiqués,
(b) des composés ayant dans la molécule au moins 2 doubles liaisons non conjuguées, éthyléniquement insaturées, en tant que réticulant, et éventuellement
(c) d'autres monomères à insaturation monoéthylénique, copolymérisables avec les monomères (a) et (b)
en présence d'initiateurs de polymérisation formant des radicaux et de 0,1 à 20 % en poids, par rapport aux monomères introduits, d'agents de surface non-ioniques, saturés, en tant que stabilisant dans des émulsions huile-dans l'eau en des quantités de 0,01 à 5 % en poids, par rapport aux émulsions.

2. Utilisation selon la revendication 1, caractérisée par le fait qu'on emploie, comme agents de surface non-ioniques saturés, des tensio-actifs non-ioniques, saturés.

3. Utilisation selon la revendication 1, caractérisée par le fait qu'on emploie, comme agents de surface non-ioniques saturés, des esters de sorbitan, des esters de saccharose ou des esters de glycérol d'acides carboxyliques en C₈-C₃₀ satures ou des produits d'alcoxylation de ces esters.

4. Utilisation selon la revendication 1, caractérisée par le fait qu'on emploie, comme agents de surface non-ioniques saturés, de la cellulose modifiée pour être rendue hydrophobe et/ou de l'amidon modifié pour être rendu hydrophobe.

5. Utilisation selon l'une des revendications 1 à 4, caractérisée par le fait qu'on soumet à la polymérisation par précipitation des mélanges de monomères de
(a) 80 à 99,99 % en poids d'acide acrylique, d'acide méthacrylique, d'acide maléique, d'anhydride d'acide maléique et/ou d'anhydride d'acide méthacrylique, et
(b) 0,01 à 20 % en poids d'éthers allyliques de pentaérythrite, de diacrylates ou de diméthacrylates de glycols ou de polyéthylèneglycols ayant des masses molaires allant jusqu'à 2000, du pentaallylsaccharose, des esters allyliques d'acide méthacrylique, des éthers allyliques de triméthylolpropane et/ou du méthylènebisacrylamide.

6. Cosmétiques ou préparations pharmaceutiques à base d'émulsions huile-dans-l'eau, caractérisés par le fait qu'ils contiennent comme stabilisant 0,01 à 5 % en poids de polymères réticulés, qui peuvent être obtenus par polymérisation par précipitation de composés monomères de
(a) des acides carboxyliques en C₃-C₈ à insaturation monoéthylénique, leurs anhydrides ou des mélanges des acides carboxyliques et des anhydrides indiqués,
(b) des composés ayant dans la molécule au moins 2 doubles liaisons non conjuguées, éthyléniquement insaturées, en tant que réticulant, et éventuellement
(c) d'autres monomères à insaturation monoéthylénique, copolymérisables avec les monomères (a) et (b)
en présence d'initiateurs de polymérisation formant des radicaux et de 0,1 à 20 % en poids, par rapport aux monomères introduits, d'agents de surface non-ioniques, saturés.
